(19) **Europäisches Patentamt**

**European Patent Office**

**Office européen des brevets**

(11) **EP 1 083 866 B1**

(12) **FASCICULE DE BREVET EUROPEEN**

(45) Date de publication et mention
de la délivrance du brevet:
**27.10.2004 Bulletin 2004/44**

(51) Int Cl.⁷: **A61K 7/06**

(21) Numéro de dépôt: **99923691.2**

(86) Numéro de dépôt international:
**PCT/FR1999/001346**

(22) Date de dépôt: **08.06.1999**

(87) Numéro de publication internationale:
**WO 1999/063954 (16.12.1999 Gazette 1999/50)**

(54) **COMPOSITION COSMETIQUE COMPRENANT AU MOINS UN POLYMERE COLLANT D'ESTER ACRYLIQUE OU METHACRYLIQUE**

Kosmetische Zusammensetzung, die mindestens ein klebriges (Meth)acrylesterpolymer enthält

COSMETIC COMPOSITION COMPRISING AT LEAST AN ACRYLIC OR METHACRYLIC ESTER TACKY POLYMER

(84) Etats contractants désignés:
**DE ES FR GB IT**

(30) Priorité: **11.06.1998 FR 9807377**

(43) Date de publication de la demande:
**21.03.2001 Bulletin 2001/12**

(73) Titulaire: **L'OREAL**
**75008 Paris (FR)**

(72) Inventeurs:
• **ROLLAT-CORVOL, Isabelle**
**F-75017 Paris (FR)**

• **SAMAIN, Henri**
**F-91570 Bièvres (FR)**

(74) Mandataire: **Bourdeau, Françoise et al**
**L'OREAL - D.I.P.I.**
**25-29 Quai Aulagnier**
**92600 Asnières (FR)**

(56) Documents cités:
**EP-A- 0 373 442       EP-A- 0 764 437**
**EP-A- 0 815 849       WO-A-94/02112**
**WO-A-97/09030         WO-A-98/00096**

**Description**

L'invention a pour objet une composition cosmétique pour les fibres kératiniques telles que les cheveux comprenant, dans un milieu cosmétiquement acceptable, au moins un polymère collant d'ester (méth)-acrylique présentant une température de transition vitreuse (Tg) comprise entre - 30 et + 20 ° C. Elle vise également un procédé de traitement des fibres kératiniques telles que les cheveux, en particulier un procédé de fixation et/ou de maintien de la coiffure, mettant en oeuvre ladite composition ainsi que l'utilisation de cette composition dans ou pour la fabrication d'une formulation cosmétique de coiffage.

Au sens de la présente invention, on entend par "fibre kératiniques", les cheveux, les cils et les sourcils et par "polymère collant", un polymère qui, après application par pression sur un polymère identique, résiste à une tentative de séparation.

La fixation de la coiffure est un élément important du coiffage qui consiste à maintenir la mise en forme déjà réalisée ou à mettre en forme les cheveux et à les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

On connaît également les gels ou les mousses de coiffage qui sont généralement appliqués sur les cheveux mouillés avant de faire un brushing ou une mise en plis. A la différence des laques aérosols classiques, ces compositions présentent l'inconvénient de ne pas permettre la fixation des cheveux dans une forme déjà réalisée. En effet, ces compositions sont essentiellement aqueuses et leur application mouille les cheveux et ne peut donc maintenir la forme initiale de la coiffure. Pour mettre en forme et fixer la coiffure, on doit donc ensuite effectuer un brushing ou un séchage.

La plupart des compositions de l'état de la technique présentent le même inconvénient de ne pas fixer ou maintenir la coiffure suffisamment durablement. Ainsi, la forme donnée initialement à la coiffure s'estompe progressivement au cours de la journée, et ceci d'autant plus vite d'ailleurs que la personne est en mouvement. En conséquence, il est souvent nécessaire de recommencer l'ensemble des opérations de coiffage et de fixation si l'on souhaite retrouver la coiffure initiale.

On recherche donc des compositions de coiffage qui procurent un effet de fixation et de maintien suffisamment forts pour que la coiffure résiste convenablement dans le temps aux diverses sollicitations.

Enfin, les compositions destinées à la fixation de la coiffure présentent parfois l'inconvénient d'altérer les propriétés cosmétiques des cheveux. Ainsi, les cheveux peuvent devenir rêches, difficiles à démêler, perdre leur toucher et leur aspect agréables. On recherche donc des compositions coiffantes procurant de bonnes propriétés cosmétiques, notamment en terme de démêlage, de douceur et de toucher.

Le document US-A-5 234 627 divulgue des compositions contenant le polymère Hycar 26120 en association avec du noir de carbone, pour la réalisation de revêtements, notamment pour les prises et les fils électriques.

Les documents US-A-4 007 147, EP-A-0 815 849, WO-A-94/01 112, EP-A-0 764 437, JP-A-62 167 307, JP-A-08 092 046 concerne, en toutes généralité, des compositions cosmétiques, sans citer d'agent de conditionnement avantageux, ni inciter une association avec un polymère de type Hycar 26120.

Il existe donc un besoin de trouver des compositions cosmétiques, notamment pour le coiffage, qui ne présentent pas l'ensemble des inconvénients indiqués ci-dessus.

De manière surprenante et inattendue, la Demanderesse a découvert que lorsque l'on met en oeuvre des polymères collants à motif ester (méth)-acrylique présentant une température de transition vitreuse (Tg) comprise entre - 30 et +20°C et un profil de décollement particulier, il est possible d'obtenir des compositions cosmétiques répondant aux exigences exprimées ci-dessus.

L'invention a donc pour objet une composition cosmétique pour les fibres kératiniques telles que les cheveux comprenant, dans un milieu cosmétiquement acceptable, au moins un polymère collant d'ester (méth)-acrylique et présentant une température de transition vitreuse comprise entre - 30 et + 20 ° C ainsi qu'un profil de décollement défini par au moins :

    (a) une force maximale de décollement Fmax > 3 Newton, et
    (b) lorsque la température Tg est inférieure à -15°C, par une énergie de séparation Es(M/V) du matériau mis en contact avec une surface en verre, inférieure à 300 µJ;

la composition comprenant, en outre, un agent de condition choisi dans le groupe comprenant les silicones non volatiles, les polymères cationiques ou amphotères et les tensioactifs cationiques.

La force maximale de décollement Fmax est la force maximale de traction, mesurée à l'aide d'un extensomètre, nécessaire pour décoller les surfaces de 38 mm$^2$, respectives de deux supports (A) et (B), rigides, inertes, non-absorbants, placés en regard l'un de l'autre ; lesdites surfaces étant enduites préalablement par le polymère sulfonique ramifié préalablement dissous à 5% dans un solvant aqueux, hydroalcoolique ou alcoolique, à raison de 1 mg/mm$^2$ , séchées pendant 24 heures à 22°C sous une humidité relative de 50%, puis soumises pendant 20 secondes à une compression de 3 Newton et enfin soumises pendant 30 secondes à une traction de vitesse 20 mm/minute.

Avantageusement, on utilise des supports (A) et (B) constitués de polyéthylène, de polypropylène, d'alliage métallique ou de verre.

L'énergie de séparation Es(M/V) est l'énergie fournie par l'extensomètre pour effectuer la séparation des surfaces respectives de 38 mm$^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) tels que définis dans la revendication 2 ou 3 et dont la surface est enduite préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50%, les deux surfaces desdits supports (C) et (D) étant soumises pendant 20 secondes à une compression de 3 Newton et enfin soumises pendant 30 secondes à une traction de vitesse 20 mm/minute.

L'énergie de séparation Es(M/V) est un travail qui peut être calculé au moyen de la formule suivante :

$$\int_{Xs1 + 0,05}^{Xs2} F(x)dx$$

où F(x) est la force nécessaire pour produire un déplacement (x) ;

xs1 est le déplacement (exprimé en millimètres) produit par la force de traction maximale ;

xs2 est le déplacement (exprimé en millimètres) produit par la force de traction permettant la séparation totale des deux surfaces des supports (C) et (D)

De préférence, la force maximale de décollement Fmax est supérieure à 4 Newton.

La concentration relative en poids en polymère collant dans la composition est en général supérieure à 0,2 %, et plus préférentiellement supérieure à 0,5%.

De manière particulièrement préférée, on choisit un polymère collant qui présente une température de transition vitreuse (Tg) supérieure à -20°C.

Les polymères collants d'ester (méth)-acrylique utilisés conformément à l'invention comprennent avantageusement:

(a) de 9 à 99% en poids d'un monomère d'ester (méth)-acrylique par rapport au poids total du polymère;

(b) jusqu'à 90% de comonomère(s);

(c) de 1 à 10 % d'un monomère vinylidène contenant un groupement carboxyle ou hydroxyle.

Le monomère d'ester (méth)-acrylique (a) répond généralement à la formule (I) ou (II):

CH2=CH-COOR (I)

CH2=C(CH3)-COOR (II)

dans lesquelles R représente un alkyle en C$_1$ à C$_{18}$, un alkoxyalkyle en C$_2$ à C$_8$, un alkylthioalkyle en C$_2$ à C$_8$ ou un cyanoalkyle en C$_2$ à C$_8$. A titre d'exemple, le monomère (a) peut être choisi dans le groupe comprenant l'acrylate de méthyle, l'acrylate d'éthyle, l'acrylate de n-butyle, l'acrylate d'isobutyle, l'acrylate d'hexyle, l'acrylate d'octyle, l'acrylate de 2-éthylhexyle, l'acrylate de décyle, le méthoxyacrylate, l'éthoxyacrylate, l'acrylate de méthylthiométhyle et l'acrylate de cyanopropyle.

Le comonomère (b) peut contenir un ou plusieurs groupes vinylidène ayant des groupes CH2=C terminaux, tels que:

- les esters acryliques ou méthacryliques, comme le méthacrylate de méthyle, le méthacrylate d'éthyle, le méthacrylate de n-butyle, l'éthacrylate de méthyle,

- les halogénures de vinyle tel que le chlorure de vinyle;

- les esters de vinyle et d'allyle tels que l'acétate de vinyle, le butyrate de vinyle, le chloroacétate de vinyle;

- les vinyles aromatiques tels que le styrène, le vinyltoluène, le chlorométhylstyrène, le vinylnaphtalène; et

- les nitriles vinyliques tels que l'acrylonitrile ou le méthacrylonitrile.

Parmi les monomères vinylidène contenant des groupement hydroxyles (c), on peut citer les monomères acrylates à groupement hydroxyle terminal, tel que l'hydroxyéthyle acrylate, l'hydroxyéthyle méthacrylate, l'hydroxypropyle acrylate, l'hydroxyéthyle méthacrylate, l'hydroxybuthyle acrylate ou encore certains dérivés hydroxyméthylés d'acrylamide diacétone, par exemple, le N-méthylol acrylamide, le N-méthylol maléamide, le N-propanolacrylamide, le N-méthylol méthacrylamide, le N-méthylol-p-vinyl benzamide.

Parmi les monomères vinylidène contenant des groupement carboxyles (c), on peut citer par exemple l'acide acrylique ou méthacrylique, l'acide itaconique, l'acide citraconique, l'acide maléique.

Les polymères collants d'ester (méth)-acrylique particulièrement visés par la présente invention sont ceux décrits dans les brevets US 5 234 627 et US 4 007 147.

Conformément à l'invention, on choisit avantageusement comme polymère d'ester (méth)-acrylique, le polymère Hycar 26 120 commercialisé par la Société Goodrich. Ce polymère Hycar 26 120 est défini par:

- une température de transition vitreuse donnée par le fournisseur égale à -10°C;
- une force maximale de décollement Fmax égale à 6,25N.

La composition peut se présenter sous forme vaporisable, de mousse, de gel ou de lotion et le véhicule cosmétiquement acceptable peut être constitué par un solvant approprié, auquel sont ajoutés des additifs tels que des agents gélifiants ou des agents moussants. En général, le solvant est choisi parmi l'eau, les alcools ou un mélange hydroalcoolique.

Les compositions peuvent contenir, en outre, une quantité appropriée de propulseurs tels que des gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non et leurs mélanges.

L'invention a également pour objet un dispositif aérosol constitué par un récipient contenant une composition aérosol constituée par d'une part une phase liquide (ou jus) contenant au moins une composition conforme à l'invention dans un solvant approprié et un propulseur ainsi qu'un moyen de distribution de ladite composition aérosol.

Encore un autre objet de l'invention est un procédé de traitement des fibres kératiniques, en particulier des cheveux, caractérisé en ce qu'on applique sur lesdites fibres la composition conforme à l'invention, avant ou après la mise en forme de la coiffure.

La composition conforme à l'invention est généralement utilisée dans ou pour la fabrication d'une formulation cosmétique de coiffage.

Les exemples ci-après permettent d'illustrer l'invention sans toutefois chercher à en limiter la portée.

EXEMPLES:

On réalise ci-après deux compositions conformes à l'invention. On estime les résultats obtenus au moyen de tests sensoriels. Les tests portent sur les propriétés cosmétiques et la tenue de la coiffure.

Exemple 1:

On prépare le composition 1 ci-après:

| HYCAR 26120 | 4g |
|---|---|
| Eau        qs | 100 g |

On introduit la composition 1 dans un flacon pompe et on l'applique sur une perruque de 20 grammes de cheveux naturels mesurant 20 centimètres.

On note un très bon maintien de la coiffure. Les cheveux sont doux et ne présentent aucune accroche lors du démêlage.

Après agitation des cheveux, on observe que la forme de la chevelure imposée avant l'application de la composition est retrouvée très rapidement.

Exemple 2:

On prépare le composition 2 ci-après comprenant un polymère collant et un agent de conditionnement:

| HYCAR 26 120 | 4g |
|---|---|
| DC 939 | 0,5g |
| Eau        qs | 100 g |

On applique la composition sur des cheveux humides, à raison de 2 g pour une perruque de 20 g de cheveux naturels mesurant 20 cm. On réalise un brushing.

On constate un très bon maintien de la coiffure et les cheveux sont très doux.

**Revendications**

1. Composition cosmétique pour les fibres kératiniques telles que les cheveux, **caractérisée par le fait qu'**elle comprend, dans un milieu cosmétiquement acceptable, au moins un polymère collant d'ester (méth)-acrylique et présentant une température de transition vitreuse comprise entre -30 et + 20 ° C ainsi qu'un profil de décollement défini par au moins:

   (a) une force maximale de décollement Fmax > 3 Newton, et
   (b) lorsque la température Tg est inférieure à -15°C, par une énergie de séparation Es(M/V) du matériau mis en contact avec une surface en verre, inférieure à 300 µJ,

   la composition comprenant en outre un agent de conditionnement choisi dans le groupe comprenant les silicones non volatiles, les polymères cationiques ou amphotères et les tensioactifs cationiques.

2. Composition selon la revendication 1, **caractérisée par le fait que** Fmax est la force maximale de traction, mesurée à l'aide d'un extensomètre, nécessaire pour décoller des surfaces de 38 mm$^2$, respectives de deux supports (A) et (B), rigides, inertes, non absorbants, placés en regard l'un de l'autre ; lesdites surfaces étant enduites préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50 %, puis soumises pendant 20 secondes à une compression de 3 Newton et enfin soumises pendant 30 secondes à une traction de vitesse 20 mm/minute.

3. Composition selon la revendication 2, **caractérisée par le fait que** les supports (A) et (B) sont constitués de polyéthylène, de polypropylène, d'alliage métallique ou de verre.

4. Composition selon la revendication 1, **caractérisée par le fait que** Es(M/V) est l'énergie fournie par l'extensomètre pour effectuer la séparation des surfaces respectives de 38 mm$^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) tels que définis dans la revendication 3 ou 4 et dont la surface est enduite préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50%, les deux surfaces desdits supports (C) et (D) étant soumises pendant 20 secondes à une compression de 3 Newton et enfin soumises pendant 30 secondes à une traction de vitesse 20 mm/minute.

5. Composition selon la revendication 4, **caractérisée par le fait que** Es(M/V) est le travail calculé au moyen de la formule suivante :

$$\int_{Xs1\,+\,0,05}^{Xs2} F(x)dx$$

      où F(x) est la force nécessaire pour produire un déplacement (x) ;

      xs1 est le déplacement (exprimé en millimètres) produit par la force de traction maximale ;

      xs2 est le déplacement (exprimé en millimètres) produit par la force de traction permettant la séparation totale des deux surfaces des supports (C) et (D).

6. Composition selon la revendication 1, **caractérisée par le fait que** la force maximale de décollement Fmax est supérieure à 4 Newton.

7. Composition selon l'une quelconque des revendications précédentes, **caractérisée par le fait que** la concentration relative en poids en polymère collant dans la composition est supérieure à 0,2 % et de préférence supérieure à 0,5 %.

8. Composition selon l'une quelconque des revendications précédentes, **caractérisée par le fait que** le polymère collant présente une température de transition vitreuse (Tg) supérieure à -20° C.

9. Composition selon l'une quelconque des revendications précédentes, **caractérisée par le fait que** le polymère collant d'ester (méth)-acrylique comprend avantageusement:

      (a) de 9 à 99 % en poids d'un monomère d'ester (méth)-acrylique par rapport au poids total du polymère;
      (b) jusqu'à 90 % de comonomère(s);
      (c) de 1 à 10 % d'un monomère vinylidène contenant un groupement carboxyle ou hydroxyle.

10. Composition selon la revendication 9, **caractérisé par le fait que** le monomère d'ester (méth)-acrylique (a) répond à la formule (I) ou (II):

$$CH2=CH-COOR \qquad (I)$$

$$CH2=C(CH3)-COOR \qquad (II)$$

dans lesquelles R représente un alkyle en C, à C$_{18}$, un alkoxyalkyle en C$_2$ à C$_8$, un alkylthioalkyle en C$_2$ à C$_8$ ou un cyanoalkyle en C$_2$ à C$_8$.

11. Composition selon la revendication 11, **caractérisée par le fait que** le comonomère (b) contient un ou plusieurs groupes vinylidène ayant des groupes CH2=C terminaux.

12. Composition selon la revendication 9, **caractérisée par le fait que** les monomères vinylidène (c) sont choisis dans le groupe comprenant l'hydroxyéthyle acrylate, l'hydroxyéthyle méthacrylate, l'hydroxypropyle acrylate, l'hydroxyéthyle méthacrylate, l'hydroxybuthyle acrylate, le N-méthylol, acrylamide, le N-méthylol maléamide, le N-propanolacrylamide, le N-méthylol méthacrylamide, le N-méthylol-p-vinyl benzamide, l'acide acrylique ou méthacrylique, l'acide itaconique, l'acide citraconique et l'acide maléique.

13. Composition selon l'une quelconque des revendications précédentes, **caractérisée par le fait que** le polymère collant se présente sous la forme de latex.

14. Composition selon l'une quelconque des revendications précédentes, **caractérisée par le fait que** le polymère collant présente:

- une température de transition vitreuse égale à -10° C, et
- une force maximale de décollement Fmax égale à 6,25N.

15. Composition selon l'une quelconque des revendications précédentes, **caractérisée en ce qu'**elle se présente sous la forme d'une composition vaporisable, de mousse, de gel ou de lotion.

16. Composition selon l'une quelconque des revendications précédentes, **caractérisée en ce que** le véhicule cosmétiquement acceptable est constitué par un solvant approprié, auquel peuvent être ajoutés des additifs tels que des agents gélifiants ou des agents moussants.

17. Composition selon l'une quelconque des revendications précédentes, **caractérisée en ce qu'**elle comprend un solvant choisi parmi l'eau, un alcool ou un mélange hydroalcoolique.

18. Composition selon l'une quelconque des revendications précédentes, **caractérisée en ce qu'**elle comprend en outre une quantité appropriée de propulseur constitué par les gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non, et leurs mélanges.

19. Dispositif aérosol constitué par un récipient contenant une composition aérosol constituée par d'une part une phase liquide (ou jus) contenant au moins une composition selon l'une quelconque des revendications 1 à 17 dans un solvant approprié et un propulseur ainsi qu'un moyen de distribution de ladite composition aérosol.

20. Procédé de traitement des fibres kératiniques, en particulier des cheveux, **caractérisé en ce qu'**on applique sur lesdites fibres la composition telle que définie dans les revendications 1 à 17, avant ou après la mise en forme de la coiffure.

21. Utilisation d'une composition selon l'une quelconque des revendications 1 à 17 dans ou pour la fabrication d'une formulation cosmétique de coiffage.

**Claims**

1. Cosmetic composition for keratinous fibres such as the hair: **characterized in that** it comprises, in a cosmetically acceptable medium, at least one tacky (meth)acrylic ester polymer having a glass transition temperature of between -30 and +20°C and a peeling profile defined by at least:

   a) a maximum peeling force Fmax > 3 Newton, and
   b) when the temperature Tg is less than -15°C, by an energy for separation Es(M/V) of the material brought into contact with a glass surface of less than 300 µJ,

   the composition additionally comprising a conditioning agent chosen from the group comprising nonvolatile silicones, cationic or amphoteric polymers and cationic surfactants.

2. Composition according to Claim 1, **characterized in that** Fmax is the maximum tensile force, measured with the aid of an extensometer, necessary to peel apart the respective 38 mm$^2$ surfaces of two rigid, inert and nonabsorbent supports (A) and (B) placed opposite each other; the said surfaces being previously coated with the tacky polymer previously dissolved at 5% in an aqueous, aqueous-alcoholic or alcoholic solvent, at the rate of 1 mg/mm$^2$, dried for 24 hours at 22°C under a relative humidity of 50%, then subjected for 20 seconds to a compression of 3 Newton and finally subjected for 30 seconds to pulling at the rate of 20 mm/min.

3. Composition according to Claim 2, **characterized in that** the supports (A) and (B) consist of polyethylene, polypropylene, metal alloy or glass.

4. Composition according to Claim 1, **characterized in that** Es(M/V) is the energy provided by the extensometer in order to carry out the separation of the respective 38 mm$^2$ surfaces of two rigid, inert and nonabsorbent supports (C) and (D) placed opposite each other; one of the said supports consisting of cut glass and the other of the said supports being of an identical nature to the supports (A) and (B) as defined in Claim 3 or 4 and whose surface is previously coated with the tacky polymer previously dissolved at 5% in an aqueous, aqueous-alcoholic or alcoholic solvent, at the rate of 1 mg/mm$^2$, dried for 24 hours at 22°C under a relative humidity of 50%, the two surfaces of the said supports (C) and (D) being subjected for 20 seconds to a compression of 3 Newton and finally subjected for 30 seconds to pulling at the rate of 20 mm/min.

5. Composition according to Claim 4, **characterized in that** Es(M/V) is the work calculated by means of the following formula:

$$\int_{Xs1+0.05}^{Xs2} F(x)dx$$

where F(x) is the force necessary to produce a movement (x);

xs1 is the movement (expressed in millimetres) produced by the maximum tensile force;

xs2 is the movement (expressed in millimetres) produced by the tensile force which allows the complete separation of the two surfaces of the supports (C) and (D).

6. Composition according to Claim 1, **characterized in that** the maximum peeling force Fmax is greater than 4 Newton.

7. Composition according to any one of the preceding claims, **characterized in that** the relative concentration by weight of tacky polymer in the composition is greater than 0.2% and preferably greater than 0.5%.

8. Composition according to any one of the preceding claims, **characterized in that** the tacky polymer has a glass transition temperature (Tg) greater than -20°C.

9. Composition according to any one of the preceding claims, **characterized in that** the tacky (meth)-acrylic ester polymer advantageously comprises:

(a) from 9 to 99% by weight of a (meth)acrylic ester monomer relative to the total weight of the polymer;
(b) up to 90% of comonomer(s);
(c) from 1 to 10% of a vinylidene monomer containing a carboxyl or hydroxyl group.

10. Composition according to Claim 9, **characterized in that** the (meth)acrylic ester monomer (a) corresponds to formula (I) or (II):

$$CH2=CH-COOR \qquad (I)$$

$$CH2=C(CH3)COOR \qquad (II)$$

in which R represents a $C_1$ to $C_{18}$ alkyl, a $C_2$ to $C_8$ alkoxyalkyl, a $C_2$ to $C_8$ alkylthioalkyl or a $C_2$ to $C_8$ cyanoalkyl.

11. Composition according to Claim 9, **characterized in that** the comonomer (b) contains one or more vinylidene groups having terminal CH2=C groups.

12. Composition according to Claim 9, **characterized in that** the vinylidene monomers (c) are chosen from the group comprising hydroxyethyl acrylate, hydroxyethyl methacrylate, hydroxypropyl acrylate, hydroxypropyl methacrylate, hydroxybutyl acrylate, N-methylolacrylamide, N-methylolmaleamide, N-propanolacrylamide, N-methylol methacrylamide, N-methylol-p-vinylbenzamide, acrylic or methacrylic acid, itaconic acid, citraconic acid and maleic acid.

13. Composition according to any one of the preceding claims, **characterized in that** the tacky polymer is provided in latex form.

14. Composition according to any one of the preceding claims, **characterized in that** the tacky polymer has:

- a glass transition temperature equal to -10°C, and
- a maximum peeling force Fmax equal to 6.25 N.

15. Composition according to any one of the preceding claims, **characterized in that** it is provided in the form of a vaporizable composition, a foam, a gel or a lotion.

16. Composition according to any one of the preceding claims, **characterized in that** the cosmetically acceptable vehicle consists of an appropriate solvent, to which additives such as gelling agents or foaming agents may be added.

17. Composition according to any one of the preceding claims, **characterized in that** it comprises a solvent chosen from water, an alcohol or an aqueous-alcoholic mixture.

18. Composition according to any one of the preceding claims, **characterized in that** it comprises, in addition, an appropriate quantity of propellant consisting of customary compressed or liquefied gases, preferably compressed air, carbon dioxide or nitrogen, or alternatively a gas which is soluble or otherwise in the composition, such as dimethyl ether, hydrocarbons which are fluorinated or otherwise and mixtures thereof.

19. Aerosol device consisting of a container containing an aerosol composition consisting, on the one hand, of a liquid phase (or juice) containing at least one composition in accordance with any one of Claims 1 to 17 in an appropriate solvent and a propellant as well as a means of distributing the said aerosol composition.

20. Method of treating keratinous fibres, in particular hair, **characterized in that** the composition as defined in Claims 1 to 17 is applied to the said fibres before or after shaping the hairstyle.

21. Use of a composition according to any one of Claims 1 to 17 in or for making a cosmetic hairstyling formulation.

## Patentansprüche

1. Kosmetische Zusammensetzung für Keratinfasern, wie z.B. für die Haare, **dadurch gekennzeichnet, dass** sie einem kosmetisch akzeptablen Medium mindestens ein klebriges (Meth)acrylesterpolymer enthält, das eine Glasübergangstemperatur von -30 bis +20 °C aufweist und ein Ablösungsprofil besitzt, das definiert wird durch zumindest:

   (a) eine maximale Ablösekraft Fmax > 3 Newton, und
   (b) wenn die Temperatur Tg unter -15 °C liegt, durch eine Trennenergie Es(M/V) des mit einer Glasoberfläche in Kontakt stehenden Materials unter 300 μJ,

   wobei die Zusammensetzung ferner ein Konditioniermittel enthält, das unter den nicht flüchtigen Siliconen, kationischen oder amphoteren Polymeren und kationischen grenzflächenaktiven Stoffen ausgewählt ist.

2. Zusammensetzung nach Anspruch 1, **dadurch gekennzeichnet, dass** Fmax die maximale Zugkraft ist, die erforderlich ist, um die Oberflächen von zwei starren, inerten und nicht absorbierenden Trägern (A) bzw. (B) mit einer Größe von 38 mm², die gegenüberliegend angeordnet sind, abzulösen; wobei die Oberflächen vorab mit dem klebrigen Polymer, das zuvor in einer Konzentration von 5 % in einem wässrigen, wässrig-alkoholischen oder alkoholischen Lösungsmittel gelöst wurde, in einer Menge von 1 mg/mm² beschichtet, 24 Stunden bei 22 °C unter einer relativen Feuchte von 50 % getrocknet und dann 20 Sekunden einem Druck von 3 Newton und schließlich 30 Sekunden einer Zuggeschwindigkeit von 20 mm/Minute ausgesetzt wurden.

3. Zusammensetzung nach Anspruch 2, **dadurch gekennzeichnet, dass** die Träger (A) und (B) aus Polyethylen, Polypropylen, einer Metalllegierung oder Glas bestehen.

4. Zusammensetzung nach Anspruch 1, **dadurch gekennzeichnet, dass** Es(M/V) die Energie ist, die von dem Extensometer aufgebracht wird, um die Trennung der Oberflächen von 2 starren, inerten und nicht absorbierenden Trägern (C) bzw. (D) von jeweils 38 mm² zu bewirken, die gegenüberliegend angeordnet sind; wobei ein Träger aus glattem Glas besteht und der andere Träger ein zu den in Ansprüchen 2 oder 3 definierten Trägern (A) und (B) gleichwertiger Träger ist, dessen Oberfläche vorab in einer Menge von 1 mm/mm² mit dem klebrigen Polymer, das zuvor in einer Menge von 5 % in einem wässrigen, wässrig-alkoholischen oder alkoholischen Lösungsmittel gelöst wurde, beschichtet wurde, wobei die Träger 24 Stunden bei 22 °C unter einer relativen Feuchte von 50 % getrocknet wurden und die beiden Oberflächen der Träger (C) und (D) während 20 Sekunden einem Druck von 3 Newton und anschließend 30 Sekunden einer Zuggeschwindigkeit von 20 mm/Minute ausgesetzt wurden.

5. Zusammensetzung nach Anspruch 4, **dadurch gekennzeichnet, dass** Es(M/V) die Arbeit ist, die mit der folgenden Formel berechnet wird:

$$\int_{Xs1+0,05}^{Xs2} F(x)dx$$

   wobei F(x) die Kraft ist, die erforderlich ist, damit eine Verschiebung (x) erzeugt wird;
   xs 1 die Verschiebung (ausgedrückt in Millimetern) ist, die durch die maximale Zugkraft bewirkt wird;
   xs2 die Verschiebung (ausgedrückt in Millimetern) ist, die durch die Zugkraft bewirkt wird, die zur vollständigen Trennung der beiden Oberflächen der Träger (C) und (D) führt.

6. Zusammensetzung nach Anspruch 1, **dadurch gekennzeichnet, dass** die maximale Ablösekraft Fmax über 4 Newton liegt.

7. Zusammensetzung nach einem der vorhergehenden Ansprüche, **dadurch gekennzeichnet, dass** die auf das Gewicht bezogene, relative Konzentration des klebrigen Polymers in der Zusammensetzung über 0,2 % und vorzugsweise über 0,5 % liegt.

8. Zusammensetzung nach einem der vorhergehenden Ansprüche, **dadurch gekennzeichnet**, das das klebrige Polymer eine Glasübergangstemperatur (Tg) über -20 °C aufweist.

9. Zusammensetzung nach einem der vorhergehenden Ansprüche, **dadurch gekennzeichnet, dass** das klebrige (Meth)acrylesterpolymer vorteilhaft umfasst:

   (a) 9 bis 99 Gew.-% (Meth)acrylestermonomer, bezogen auf das Gesamtgewicht des Polymers;
   (b) bis zu 90 % Comonomer(e);
   (c) 1 bis 10 °% Vinylidenmonomer, das eine Carboxy- oder Hydroxygruppe enthält.

**10.** Zusammensetzung nach Anspruch 9, **dadurch gekennzeichnet, dass** das (Meth)acrylestermonomer (a) der Formel (I) oder (II) entspricht:

$$CH_2=CH-COOR \qquad (I)$$

$$CH_2=C(CH_3)-COOR \qquad (II)$$

worin R $C_{1-18}$-Alkyl, $C_{2-8}$-Alkoxyalkyl, $C_{2-8}$-Alkylthioalkyl oder $C_{2-8}$-Cyanoalkyl bedeutet.

**11.** Zusammensetzung nach Anspruch 10, **dadurch gekennzeichnet**, das das Comonomer (b) eine oder mehrere Vinylidengruppen enthält, die endständige $CH_2=C$-Gruppen aufweisen.

**12.** Zusammensetzung nach Anspruch 9, **dadurch gekennzeichnet, dass** die Vinylidenmonomere (c) unter Hydroxyethylacrylat, Hydroxymethacrylat, Hydroxypropylacrylat, Hydroxyethylmethacrylat, Hydroxybutylacrylat, N-Methylolacrylamid, N-Methylolmaleamid, N-Propanolacrylamid, N-Methylolmethacrylamid, N-Methyl-p-vinylbenzamid, Acrylsäure, Methacrylsäure, Itaconsäure, Citraconsäure und Maleinsäure ausgewählt ist.

**13.** Zusammensetzung nach einem der vorhergehenden Ansprüche, **dadurch gekennzeichnet, dass** das klebrige Polymer in Form von Latex vorliegt.

**14.** Zusammensetzung nach einem der vorhergehenden Ansprüche, **dadurch gekennzeichnet, dass** das klebrige Polymer aufweist:

- eine Glasübergangstemperatur von -10 °C, und
- eine maximale Ablösekraft Fmax von 6,25 N.

**15.** Zusammensetzung nach einem der vorhergehenden Ansprüche, **dadurch gekennzeichnet, dass** sie als zerstäubbare Zusammensetzung, Schaum, Gel oder Lotion vorliegt.

**16.** Zusammensetzung nach einem der vorhergehenden Ansprüche, **dadurch gekennzeichnet, dass** der kosmetisch akzeptable Träger aus einem geeigneten Lösungsmittel besteht, in das Zusätze gegeben werden, wie Gelbildner oder Schaummittel.

**17.** Zusammensetzung nach einem der vorhergehenden Ansprüche, **dadurch gekennzeichnet, dass** sie ein Lösungsmittel enthält, das unter Wasser, Alkoholen oder wässrig-alkoholischen Gemischen ausgewählt ist.

**18.** Zusammensetzung nach einem der vorhergehenden Ansprüche, **dadurch gekennzeichnet, dass** sie eine geeignete Menge eines Treibmittels enthält, das aus üblichen komprimierten oder verflüssigten Gasen, vorzugsweise komprimierter Luft, komprimiertem Kohlendioxid oder komprimiertem Stickstoff, oder in der Zusammensetzung löslichen oder nicht löslichen Gasen, wie Dimethylether, fluorierten oder nicht fluorierten Kohlenwasserstoffen und deren Gemischen besteht.

**19.** Aerosolvorrichtung, die aus einem Behälter mit einer Aerosolzusammensetzung, die aus einer flüssigen Phase (oder Flüssigkeit) mit mindestens einer Zusammensetzung nach einem der Ansprüche 1 bis 17 in einem geeigneten Lösungsmittel und einem Treibmittel besteht, sowie aus einer Einrichtung zur Verteilung der Aerosolzusammensetzung besteht.

**20.** Verfahren zur Behandlung von Keratinfasern und insbesondere zur Behandlung der Haare, **dadurch gekennzeichnet, dass** auf die Fasern die Zusammensetzung nach einem der Ansprüche 1 bis 17 aufgebracht wird, bevor die Frisur in Form gebracht wird oder nachdem sie geformt wurde.

**21.** Verwendung einer Zusammensetzung nach einem der Ansprüche 1 bis 17 für die Herstellung einer kosmetischen Formulierung zur Frisurengestaltung oder in einer solchen Formulierung.